(19) Europäisches Patentamt
European Patent Office
Office européen des brevets

(11) Publication number: **0 127 469 A2**

# (12) EUROPEAN PATENT APPLICATION

(21) Application number: 84303561.9

(22) Date of filing: 25.05.84

(51) Int. Cl.³: C 07 C 143/13
C 07 C 143/833, C 07 D 333/38
A 01 N 47/24, A 01 N 25/32

(30) Priority: 26.05.83 US 498471

(43) Date of publication of application: 05.12.84 Bulletin 84/49

(84) Designated Contracting States: AT BE CH DE FR GB IT LI LU NL SE

(71) Applicant: E.I. DU PONT DE NEMOURS AND COMPANY
1007 Market Street
Wilmington Delaware 19898(US)

(72) Inventor: Amuti, Kofi Sam
Apt. 46 5504 Limerick Circle
Wilmington Delaware 19808(US)

(72) Inventor: Levitt, George
3218 Romilly Road
Wilmington Delaware 19810(US)

(74) Representative: Hildyard, Edward Martin et al,
Frank B. Dehn & Co. European Patent Attorneys Imperial House 15-19 Kingsway
London WC2B 6UZ(GB)

## (54) Antidotes for sulfonylurea herbicides.

(57) Esters of the formulae

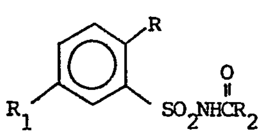

and

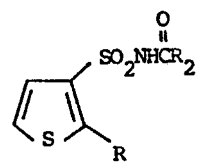

(I) (II)

and their agriculturally suitable salts,
where

R is $CO_2CH_3$ or $CO_2C_2H_5$;

$R_1$ is H, Cl or $CF_3$;

$R_2$ is $C_1$–$C_3$ alkoxy, amino or substituted amino;

are useful to mitigate damage to crops caused by sulfonylurea herbicides. The compounds may be applied in various ways, e.g. in admixture with the herbicide or as a dressing on the crop seed.

The novel compounds may be made by reacting the appropriate benzenesulfonyl or thiophenesulfonyl isocyanate with an appropriate amine or alcohol.

EP 0 127 469 A2

## Background of the Invention

The "sulfonylurea" herbicides are an extremely potent class of herbicides discovered within the last few years. A multitude of structural variations exist within this class of herbicides, but they generally consist of a sulfonylurea bridge, $-SO_2NHCONH-$, linking two aromatic or heteroaromatic rings. See, for example, the compounds disclosed in U.S. Patents 4,169,719, U.S. 4,127,405, U.S. 4,120,691, U.S. 4,221,585, U.S. 4,190,432, U.S. 4,225,337, U.S. 4,371,391, U.S. 4,339,266, U.S. 4,191,553, U.S. 4,305,884, U.S. 4,214,890, U.S. 4,339,267, U.S. 4,302,241, U.S. 4,342,587, U.S. 4,310,346, U.S. 4,293,330, U.S. 4,301,286, U.S. 4,370,479, U.S. 4,370,480, U.S. 4,368,067, U.S. 4,369,320, U.S. 4,348,219, U.S. 4,348,220, U.S. 4,333,760, U.S. 4,368,069, and U.S. 4,323,611 as well as in our EP-A-7,687.

It is difficult to find herbicidal compounds which exhibit sufficient selectivity, that is, which will control weeds but which will not damage useful crop plants. Many of the sulfonylurea herbicides exhibit excellent selectivity on a number of crops while still controlling weeds at very low application rates. Nonetheless, even with such selective herbicides, it is often desired to provide even greater protection for crop plants than that provided by the herbicide alone. Antidotes have been found for other classes of herbicides which, when applied in combination with the herbicide, act to protect the useful crop plant from damage caused by the herbicide but which do not adversely affect the action of the herbicide on weeds. See, for example, U.S. Patent 3,564,768 which discloses that 1,8-naphthalic anhydride protects

corn from damage caused by N,N-dialkylthiocarbamate ester preemergent herbicides, or U.S. 4,070,389 which discloses α-(cyanomethoxyimino)benzacetonitrile as a safener for herbicides such as the triazine and carbamate herbicides. U.S. Patent 4,343,649 discloses that these known antidotes, as well as the antidote N,N-diallyl-2,2-dichloroacetamide, are useful for protecting crops from injury caused by certain sulfonylurea herbicides, namely, 2-chloro-N-[(4-methoxy-6-methyl-1,3,5-triazin-2-yl)aminocarbonyl]benzenesulfonamide, 2,5-dichloro-N-[(4,6-dimethoxypyrimidin-2-yl)-aminocarbonyl]benzenesulfonamide and 2-[N-[(4,6-dimethylpyrimidin-2-yl)aminocarbonyl]aminosulfonyl]-benzoic acid, methyl ester. There is a continuing need for methods of antidoting the sulfonylurea herbicides and thereby increasing their usefulness to the agriculture industry.

## Summary of the Invention

It has now been found that compounds of Formulas I and II can be used to protect crops from injury caused by sulfonylurea herbicides.

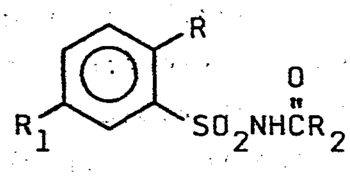

I

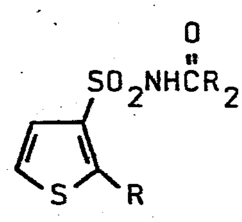

II

where

R is $CO_2CH_3$ or $CO_2C_2H_5$;

$R_1$ is H, Cl or $CF_3$;

$R_2$ is $C_1$-$C_3$ alkoxy or $NR_3R_4$;

$R_3$ is H, $OCH_3$, $C_1$-$C_4$ alkyl, cyclohexyl,

$C_1$-$C_4$ alkyl substituted with 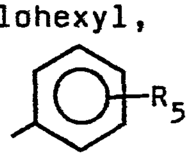,

$C_1$-$C_4$ alkyl substituted with $C_1$-$C_2$ alkoxy or $C_1$-$C_4$ alkyl substituted with ethoxyethoxy;

$R_4$ is H or $C_1$-$C_2$ alkyl;

$R_5$ is H, Cl or $OCH_3$;

and agriculturally suitable salts thereof.

This invention therefore relates to the method of using compounds of Formulas I and II to protect crop plants from injury due to herbicidal sulfonylureas. It also relates to herbicidal compositions comprising an herbicidally effective amount of one or more sulfonylurea herbicides and an antidotally effective amount of one or more compounds of Formula I or II.

The compounds of Formula II are novel. Compounds of Formula I are novel where R-$R_5$ are defined as above, provided that when R is $CO_2CH_3$ and $R_2$ is $C_1$-$C_3$ alkoxy, then $R_1$ is other than H. This invention therefore also relates to these novel compounds and their agriculturally suitable salts and to agricultural compositions containing them.

Certain of the antidotal compounds of Formulas I and II are preferred for reasons of their great effectiveness and favorable ease of synthesis. These groups of compounds are those where:

1) $R_1$ is H.

2) $R_2$ is $OCH_3$ or $NR_3R_4$.

3) $R_4$ is H and, when $R_3$ is $C_1$-$C_4$ alkyl substituted with 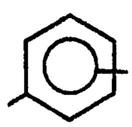 $R_5$, $R_5$ is H.

4) $R_3$ is H or $C_1$-$C_4$ alkyl optionally substituted with phenyl or with $C_1$-$C_2$ alkoxy.

Specifically preferred for the same reasons are the following antidotal compounds:

- 2-[(aminocarbonyl)aminosulfonyl]benzoic acid, methyl ester, ammonium salt;

- 2-[(aminocarbonyl)aminosulfonyl]benzoic acid, methyl ester; and
- 3-[(aminocarbonyl)aminosulfonyl]-2-thiophenecarboxylic acid, methyl ester

The antidotal compounds of Formulas I and II have been found to be especially effective in protecting crop plants from damage caused by sulfonylurea herbicides of Formula III:

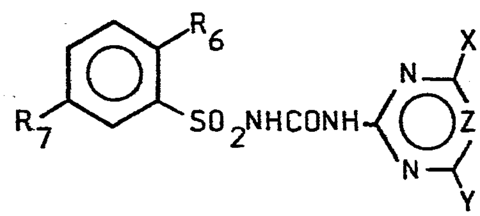

III

where

$R_6$ is $CO_2R_8$ or Cl;
$R_7$ is H, Cl or $CF_3$;
$R_8$ is $C_1$-$C_3$ alkyl;
X is $CH_3$ or $OCH_3$;
Y is $CH_3$, $OCH_3$ or Cl; and
Z is CH or N;

provided that

(a) when Y is Cl, then Z is CH; and
(b) when $R_6$ is Cl, then $R_7$ is H, X is $CH_3$, Y is $OCH_3$ and Z is N;

and their agriculturally suitable salts.

## Detailed Description

### Synthesis

Compounds of Formulas I and II are prepared as shown in Equation 1 by the reaction of an appropriately substituted o-alkoxycarbonylbenzenesulfonyl isocyanate (IV) or 2-alkoxycarbonylthiophene-3-sulfonyl isocyanate (V) with an amine or alcohol wherein R, $R_1$ and $R_2$ are as previously defined.

Equation 1

a. 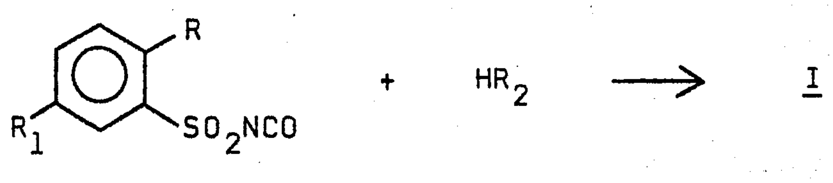

IV

b. 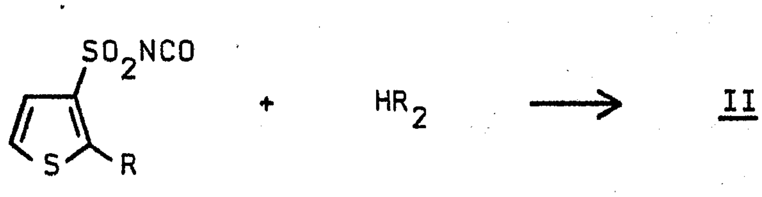

V

The reactions of Equations 1a and 1b are best carried out under anhydrous conditions in an inert aprotic solvent such as tetrahydrofuran, methylene chloride, chloroform, ethyl ether, acetonitrile or toluene. The sulfonyl isocyanate can be added neat or dissolved in the solvent and added to a solution of the appropriate amine or alcohol. A reverse addition of the reagents can be carried out where $R_2$ is $C_1$-$C_3$ alkoxy or $NR_4R_5$ wherein $R_4$ and $R_5$ are not hydrogen. Although pressure and temperature are not critical, atmospheric pressure and temperatures of from 0°C to the boiling point of the solvent or ambient temperature are suitable for these reactions. In the presence of an excess of the amine $HNR_4R_5$ in Equation 1 the amine salt of the sulfonylurea is formed. This product can be dissolved in water or a lower alcohol and acidified with an acid such as hydrochloric acid or other strong mineral acid to yield the free acid form of the sulfonylurea.

Alternatively, where $R_2$ is $C_1$-$C_3$ alkoxy, these compounds can also be prepared as described in Japan Patent 57126-404. The corresponding benzenesulfonamide is dissolved in an alkaline aqueous solution and is reacted with the appropriate alkyl chloroformate e.g. methyl chloroformate, at low temperatures to yield the desired compounds. Where $R_2$ is $NR_4R_5$ and $R_4$ is other than H or methoxy and $R_5$ is H, the compounds of this invention can also be prepared by the addition of an alkyl isocyanate to the appropriate sulfonamide as shown in Equation 2.

<u>Equation 2</u>

a.

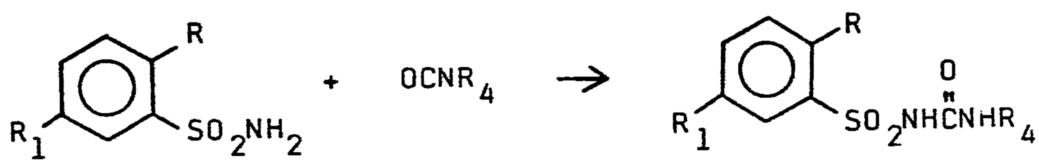

b.

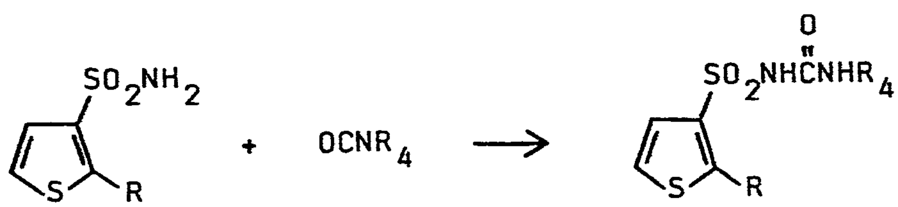

This reaction can be carried out by mixing stoichiometric amounts of the appropriate sulfonamide, isocyanate and a base such as potassium carbonate in a polar solvent such as acetone or methyl ethyl ketone at ambient to reflux temperature. The reaction mixture is poured into ice-water and neutralized with

hydrochloric or similar acid to precipitate the desired product.

The sulfonylurea herbicides of Formula III are known, and their preparation is disclosed in U.S. Patent 4,127,405 and in EP-A-7,687.

Agriculturally suitable salts of the antidotes of Formulas I and II and of the herbicides of Formula III can be prepared by methods known in the art. Suitable salts include alkali metal or alkaline earth metal salts, e.g. potassium, sodium or calcium salts; quaternary amine salts; and acid addition salts, e.g. p-toluenesulfonic acid or trichloroacetic acid addition salts.

Following are examples illustrating the preparation of the antidotal compounds of this invention. All degrees are in Centigrade.

## Example 1

Methyl 2-[(butylaminocarbonyl)aminosulfonyl]benzoate

A mixture prepared from 20.13 g of methyl 2-(aminosulfonyl)benzoate, 12.0 g of n-butyl isocyanate, 13.0 g of anhydrous potassium carbonate and 150 ml of acetone was stirred for two days at ambient temperature and pressure. It was then poured into 500 g of ice and water and neutralized to pH 7 by adding concentrated hydrochloric acid to yield a gummy precipitate. Extraction with methylene chloride (2 x 300 ml) followed by drying of the organic phase over magnesium sulfate, filtering and evaporation of the solvent yielded a gummy residue. This product crystallized slowly on standing to yield 18.4 g of the desired compound, melting at 83-88°, which was suitably pure for the purposes of this invention.

Example 2

Methyl 2-[(aminocarbonyl)aminosulfonyl]benzoate, ammonium salt

To a solution prepared from 2 ml of liquified ammonia in 30 ml of tetrahydrofuran prepared under anhydrous conditions was added 1 g of methyl 2-isocyanatosulfonylbenzoate at 0°. The resultant product was isolated by filtration and after washing with ethyl ether melted at 118° with evolution of a gas. It showed absorption peaks by infrared analysis at 1720, 1650 and 1580 $cm^{-1}$, consistent with the desired sulfonylurea product.

Example 3

Methyl 2-[(aminocarbonyl)aminosulfonyl]benzoate

Enough water was added to the ammonium salt, prepared as in Example 2, to dissolve the sample. This solution was acidified to pH 3 by the addition of 10% aqueous hydrochloric acid to precipitate the desired compound, melting at 165-170°. It showed absorption peaks by infrared absorption spectroscopy at 1760, 1690, 1550 and 1530 $cm^{-1}$, consistent with the desired product.

Example 4

Methyl 3-[(aminocarbonyl)aminosulfonyl]thiophenecarboxylate, ammonium salt

To a solution containing 3 ml of liquified ammonia in 35 ml of tetrahydrofuran under anhydrous conditions was added 3 g of methyl 3-(isocyanatosulfonyl)thiophene-2-carboxylate at -10°. The precipitate thus formed was removed by filtration and air dried. It melted at 147-149°, with evolution of gas, and showed absorption peaks by infrared absorption spectroscopy at 3380, 3200 and 3180 $cm^{-1}$, con-

sistent with NH groups and 1680, 1650 and 1575 $cm^{-1}$, consistent with the desired sulfonylurea product.

Example 5

Methyl 3-[(aminosulfonyl)aminocarbonyl]-2-thiophenecarboxylate

The ammonium salt obtained in Example 4 (0.9 grams) was dissolved in 25 ml of water and the desired free acid form of the title compound was precipitated by the addition of 10% hydrochloric acid to pH 3. The product which was removed by filtration melted at 146-149° with decomposition and showed absorption peaks by infrared absorption spectroscopy, at 3480, 3360, 1740 and 1705 $cm^{-1}$, consistent with the desired product.

By following the procedures of Examples 1-5, the compounds listed in Tables I and II can be prepared.

Table Ia

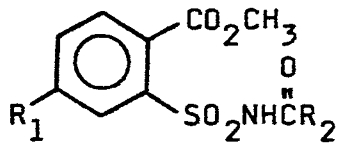

| $R_1$ | $R_2$ | m.p.(°C) |
|---|---|---|
| H | $OCH_3$ | 142-146 |
| H | $OC_2H_5$ | |
| H | $OCH_2CH_2CH_3$ | |
| H | $NHCH_3$ | 172-175 |
| H | $NHC_2H_5$ | |
| H | $NHCH_2CH_2CH_3$ | |
| H | $NHCH(CH_3)_2$•$NHCH(CH_3)$ | 170-171 |
| H | $NHCH(CH_3)(C_2H_5)$ | 114-118 |
| H | $N(CH_3)_2$ | |
| H | $NCH_3(CH_2CH_3)$ | |
| H | $N(CH_2CH_3)(CH_2CH_2CH_2CH_3)$ | |
| H | $N(CH_2CH_3)_2$ | |
| H | $NHCH_2$- 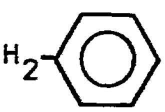 | |
| H | $NHCH_2$- 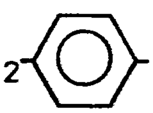 -Cl | |
| H | $NHCH_2$- 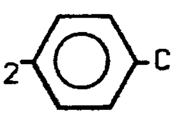 $-CH_3$ | |
| H | $NHCH_2$- 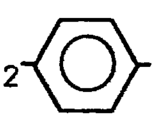 $-OCH_3$ | |
| H | $NHCH_2CH_2OCH_3$ | 73-76 |
| H | $NHCH_2CH_2OC_2H_5$ | |
| H | $NHCH_2CH_2CH_2OCH_3$ | |
| H | $NHCH_2CH_2CH_2CH_2OC_2H_5$ | |
| H | $NHCH_2CH_2OCH_2CH_2OCH_2CH_3$ | |

Table Ia (continued)

| $R_1$ | $R_2$ | m.p.(°C) |
|---|---|---|
| H | 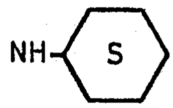 | 113-115 |
| H | $NHCH_2CH_2CH_2OCH_2CH_2OCH_2CH_3$ | |
| H | 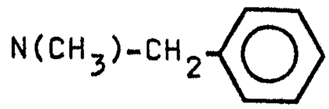 | |
| H | 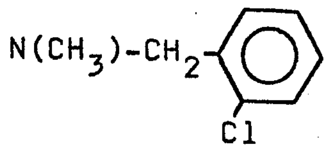 | |
| Cl | $N(OCH_3)CH_3$ | |
| Cl | $NH_2$ | |
| Cl | $NHCH_3$ | |
| Cl | $NHC_2H_5$ | |
| Cl | $NHCH(CH_3)_2$ | |
| Cl | $N(CH_3)_2$ | |
| Cl | $N(OCH_3)CH_3$ | |
| $CF_3$ | $NH_2$ | |
| $CF_3$ | $NHCH_3$ | |
| $CF_3$ | $N(CH_3)_2$ | |
| $CF_3$ | $N(CH_3)C_2H_5$ | |
| $CF_3$ | $NHCH(CH_3)_2$ | |
| $CF_3$ | $NHCH_2CH_2CH_2CH_3$ | |
| Cl | $OCH_3$ | |
| Cl | $OCH(CH_3)_2$ | |
| $CF_3$ | $OCH_3$ | |
| $CF_3$ | $OCH_2CH_2CH_3$ | |

Table Ib

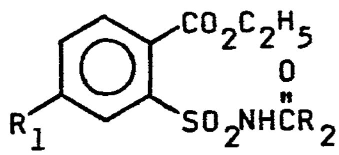

| $R_1$ | $R_2$ | m.p.(°C) |
|---|---|---|
| H | $OCH_3$ | |
| H | $OC_2H_5$ | |
| H | $OCH_2CH_2CH_3$ | |
| H | $NH_2$ | 121-122 |
| H | $NHCH_3$ | |
| H | $NHC_2H_5$ | |
| H | $NHCH_2CH_2CH_3$ | |
| H | $NHCH(CH_3)_2$ | |
| H | $NHCH(CH_3)(C_2H_5)$ | |
| H | $N(CH_3)_2$ | |
| H | $NCH_3(CH_2CH_3)$ | |
| H | $N(CH_2CH_3)(CH_2CH_2CH_2CH_3)$ | |
| H | $N(CH_2CH_3)_2$ | |
| H | 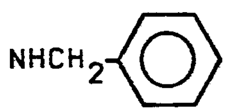 | |
| H | 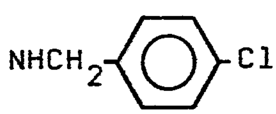 | |
| H | 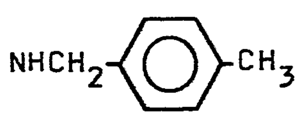 | |
| H | 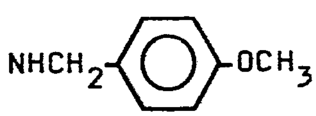 | |
| H | $NHCH_2CH_2OCH_3$ | |
| H | $NHCH_2CH_2OC_2H_5$ | |
| H | $NHCH_2CH_2CH_2OCH_3$ | |
| H | $NHCH_2CH_2CH_2CH_2OC_2H_5$ | |

Table Ib (continued)

| $R_1$ | $R_2$ | m.p.(°C) |
|---|---|---|
| H | $N(CH_3)$- 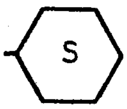 | |
| H | $NHCH_2CH_2OCH_2CH_2OCH_2CH_3$ | |
| H | $NHCH_2CH_2CH_2OCH_2CH_2OCH_2CH_3$ | |
| H | $N(CH_3)-CH_2$- 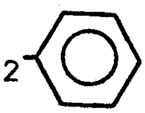 | |
| H | $N(CH_3)-CH_2$- 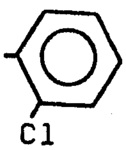 | |
| Cl | $N(OCH_3)CH_3$ | |
| Cl | $NH_2$ | |
| Cl | $NHCH_3$ | |
| Cl | $NHC_2H_5$ | |
| Cl | $NHCH(CH_3)_2$ | |
| Cl | $N(CH_3)_2$ | |
| Cl | $N(OCH_3)CH_3$ | |
| $CF_3$ | $NH_2$ | |
| $CF_3$ | $NHCH_3$ | |
| $CF_3$ | $N(CH_3)_2$ | |
| $CF_3$ | $N(CH_3)C_2H_5$ | |
| $CF_3$ | $NHCH(CH_3)_2$ | |
| $CF_3$ | $NHCH_2CH_2CH_2CH_3$ | |
| Cl | $OCH_3$ | |
| Cl | $OCH_2CH_2CH_3$ | |
| $CF_3$ | $OC_2H_5$ | |
| $CF_3$ | $OCH(CH_3)_2$ | |
| H | $NHOCH_3$ | wax |

Table IIa

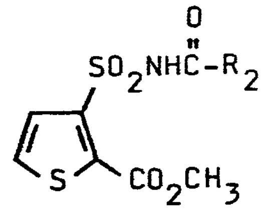

| $R_2$ |
|---|
| $OCH_3$ |
| $OC_2H_5$ |
| $OCH_2CH_2CH_3$ |
| $NHCH_3$ |
| $NHC_2H_5$ |
| $NHCH_2CH_2CH_3$ |
| $NHCH(CH_3)_2$ |
| $NHCH(CH_3)(C_2H_5)$ |
| $N(CH_3)_2$ |
| $NCH_3(CH_2CH_3)$ |
| $N(CH_2CH_3)(CH_2CH_2CH_2CH_3)$ |
| $N(CH_2CH_3)_2$ |
| 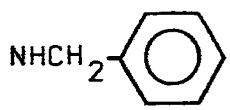 |
| 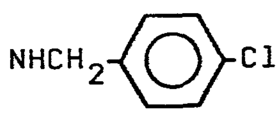 |
| 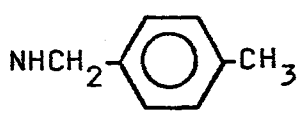 |
| 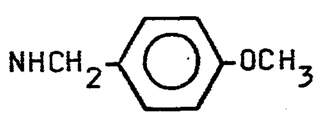 |
| $NHCH_2CH_2OCH_3$ |
| $NHCH_2CH_2OC_2H_5$ |
| $NHCH_2CH_2CH_2OCH_3$ |

IIa (continued)

$\underline{R_2}$

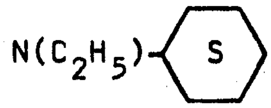

$NHCH_2CH_2CH_2CH_2OC_2H_5$

$NHCH_2CH_2OCH_2CH_2OCH_2CH_3$

$NHCH_2CH_2CH_2OCH_2CH_2OCH_2CH_3$

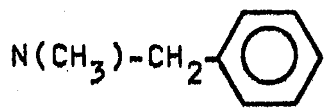

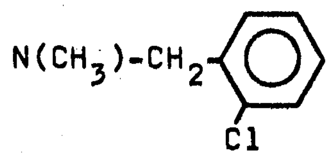

$N(OCH_3)CH_3$

Table IIb

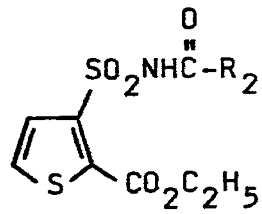

| $R_2$ |
| --- |
| $OCH_3$ |
| $OC_2H_5$ |
| $OCH_2CH_2CH_3$ |
| $NH_2$ |
| $NHCH_3$ |
| $NHC_2H_5$ |
| $NHCH_2CH_2CH_3$ |
| $NHCH(CH_3)_2$ |
| $NHCH(CH_3)(C_2H_5)$ |
| $N(CH_3)_2$ |
| $NCH_3(CH_2CH_3)$ |
| $N(CH_2CH_3)(CH_2CH_2CH_2CH_3)$ |
| $N(CH_2CH_3)_2$ |
| 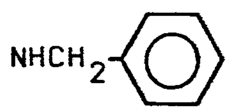 |
| 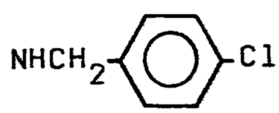 |
| 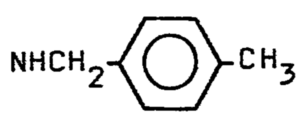 |
| 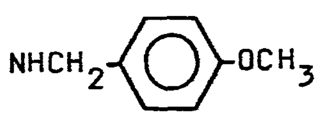 |
| $NHCH_2CH_2OCH_3$ |
| $NHCH_2CH_2OC_2H_5$ |
| $NHCH_2CH_2CH_2OCH_3$ |

IIb (continued)

$\underline{R_2}$

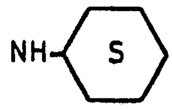

$NHCH_2CH_2CH_2CH_2OC_2H_5$

$NHCH_2CH_2OCH_2CH_2OCH_2CH_3$

$NHCH_2CH_2CH_2OCH_2CH_2OCH_2CH_3$

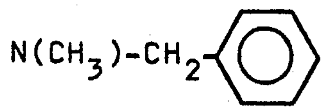

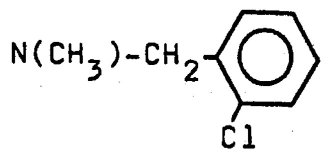

$N(OCH_3)CH_3$

Formulation

The antidotes described herein can be formulated in a number of ways:

(a) the antidote can be formulated for application directly to the crop seed;

(b) the antidote and herbicide may be formulated separately and applied separately or applied simultaneously in an appropriate weight ratio, e.g., as a tank mix; or

(c) the antidote and herbicide may be formulated together in the proper weight ratio.

Formulations of the compounds of Formulas I and II can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few litres to several thousand litres per hectare (from a few pints to several hundred gallons per acre). High strength compositions are primarily used as intermediates for further formulation. The formulations, broadly, contain about 1% to 99% by weight of active ingredient(s) and at least one of (a) about 0.1% to 20% surfactant(s) and (b) about 5% to 99% solid or liquid diluent(s). More specifically, they will contain these ingredients in the following approximate proportions:

| | Weight Percent | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 5-50 | 40-95 | 0-15 |
| Aqueous Suspension, Solution | 10-50 | 40-84 | 1-20 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Co., Glen Rock, New Jersey, as well as Sisely and Wood, "Encyclopedia of Surface Active Agents", Chemical Publishing Co., Inc., New York, 1964, list surfactants and recommended uses. All formulations can contain minor amounts of additives to

reduce foam, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 4th Ed., McGraw-Hill, New York, 1973, pp. 8-59ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

The following examples are illustrative of the formulations suitable for this invention.

Formulation Examples - Herbicidal Antidotes

Example 6

Antidote Alone

Wettable Powder

| | |
|---|---|
| 2-[(aminocarbonyl)aminosulfonyl]benzoic acid, methyl ester | 40% |
| dioctyl sodium sulfosuccinate | 1.5% |
| sodium ligninsulfonate | 3% |
| low viscosity methyl cellulose | 1.5% |
| attapulgite | 54% |

The ingredients are thoroughly blended, passed through an air mill to produce an average particle size under 15 microns, reblended, and sifted through a U.S.S. No. 50 sieve (0.3 mm opening) before packaging.

Example 7

Antidote Plus Herbicide

Wettable Powder

| | |
|---|---|
| 2-chloro-N-[[(4-methoxy-6-methyl-1,3,5-triazin-2-yl)-amino]carbonyl]benzenesulfonamide | 3% |
| 2-[(aminocarbonyl)aminosulfonyl]benzoic acid, methyl ester | 50% |
| dioctyl sodium sulfosuccinate | 1.5% |
| sodium liginsulfonate | 3% |
| low viscosity methyl cellulose | 1.5% |
| attapulgite | 41% |

The ingredients are thoroughly blended, passed through an air mill to produce an average particle size under 15 microns, reblended, and sifted through a U.S.S. No. 50 sieve (0.3 mm opening) before packaging.

Example 8

Antidote Alone

Aqueous Suspension

| | |
|---|---|
| 3-[(aminocarbonyl)aminosulfonyl]-2-thiophenecarboxylic acid, methyl ester | 25% |
| hydrated attapulgite | 3% |
| crude calcium lignosulfonate | 10% |
| sodium dihydrogen phosphate | 0.5% |
| water | 61.5% |

The ingredients are ground together in a sand mill until the solid particles have been reduced to diameters under 5 microns.

Example 9

Antidote Plus Herbicide

Aqueous Suspension

| | |
|---|---|
| 2-[N-[(4,6-dimethylpyrimidin-2-yl)aminocarbonyl]-aminosulfonyl]benzoic acid, methyl ester | 5% |
| 3-[(aminocarbonyl)aminosulfonyl]-2-thiophenecarboxylic acid, methyl ester | 25% |
| hydrated attapulgite | 3% |
| crude calcium lignosulfonate | 10% |
| sodium hydrogen phosphate | 0.5% |
| water | 56.5% |

The ingredients are ground together in a sand mill until the solid particles have been reduced to diameters under 5 microns.

Example 10

Dust Seed Coat

| | |
|---|---|
| 2-[(aminocarbonyl)aminosulfonyl]benzoic acid, methyl ester, ammonium salt | 75% |
| permanent red 2B, calcium salt, extended on Blanc Fixe | 5% |
| diatomaceous earth | 20% |

The ingredients are blended, coarsely hammer-milled and passed through a fluid mill to produce particles of active ingredient that are essentially below 10 microns in diameter. The product is reblended before packaging.

Utility

Test results indicate that compounds of Formulas I and II are useful in protecting crop plants from injury due to sulfonylurea herbicides by combining application of the herbicide to the locus of the plant with application of a non-phytotoxic antidotally effective amount of one or more of the compounds of Formulas I or II to the crop seed, the crop plant or to the locus of the crop plant. Crop plants which can be protected from injury include cereal crops such as corn, wheat, sorghum and barley. Soybean crops can also be protected by means of this invention.

A number of methods are available for combining application of the herbicidal and antidotal compounds described herein. The herbicide and antidote may be applied preemergence (applied to the soil after planting, but before crop plants emerge), preplant soil incorporated (applied to the soil and mixed into the soil before planting), or postemergence (applied to the emerged crop and/or weeds and on the exposed soil surface). Postemergence treatments may be directed so that the herbicide and/or antidote is applied primarily to the weeds or crop.

The antidotes and herbicides may be applied either simultaneously or sequentially. If applied sequentially, the antidote application usually precedes the herbicide application, although the reverse sequence can also be effective. In addition, the antidote may be applied directly to the crop seed. The seeds may be uniformly coated with the antidote

according to standard seed treating procedures prior to planting. Alternatively, the antidote may be applied over the exposed seed in open furrow at planting, just prior to covering the seed with soil (in-the-furrow treatment).

The ratio of antidote to herbicide used will vary, depending upon the specific antidote and herbicide used and upon the method of application used. The crop species and cultural practices may also have an effect. For example, when the antidote is applied as a seed coating, it may be applied at a rate of about 0.1 to 0.5% of the seed weight. When the seeds are planted at from 10 to 150 kg/ha, the antidote would be distributed at from 0.01 to 0.75 kg/ha. In the preemergence, preplant soil incorporation and postemergence treatments, the antidote may be applied at about 0.1 to 10 kg/ha. Similar rates may be used for in-the-row treatments; however, since only the furrow is treated, the lower per hectare rate would be diminished to as low as about 0.01 kg/ha, depending upon the row spacing. Since the herbicides may be used at rates of about 0.001 to 2 kg/ha, the ratio of antidote to herbicide may vary between 1:200 to 10,000:1. One skilled in the art could determine the proper ratio to use in a given situation.

In the following tests, the herbicidal and antidotal compounds are referred to by numbers as follows:

## Antidotes Tested

Compound 1

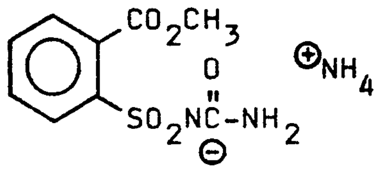

Compound 2

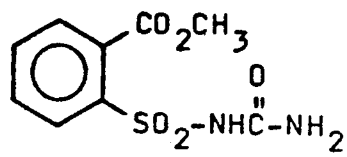

Compound 3

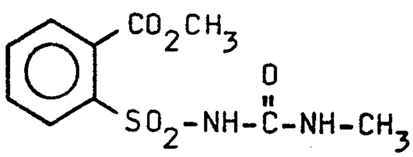

Compound 4

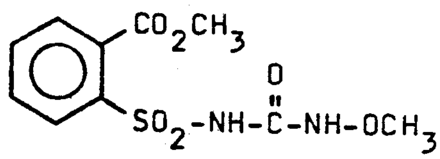

Compound 5

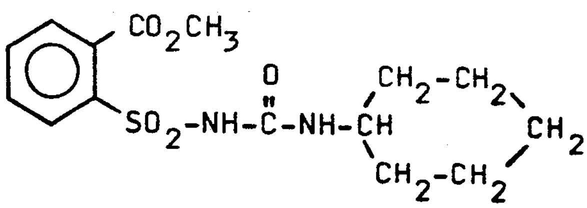

Compound 6

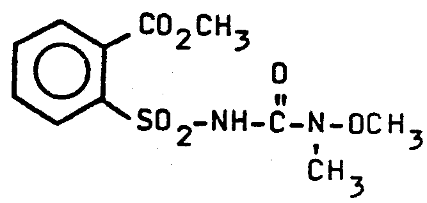

Compound 7

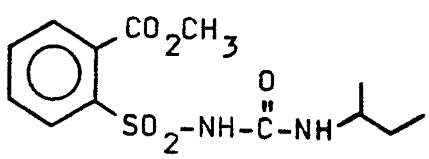

Compound 8

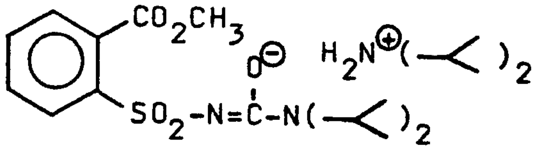

Antidotes Tested (continued)

Compound 9

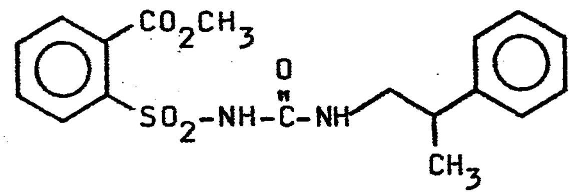

Compound 10

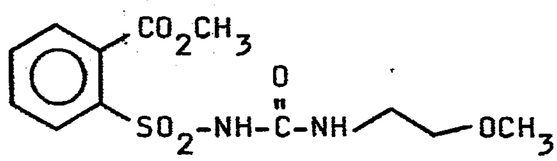

Compound 11

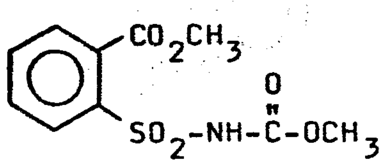

Compound 12

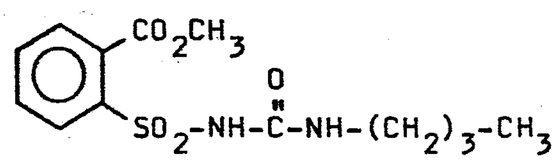

Compound 13

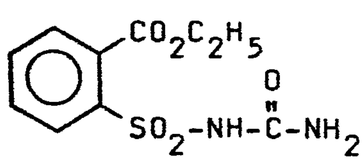

Compound 14

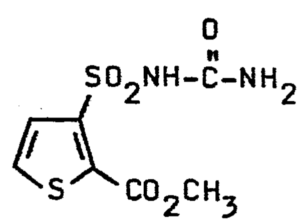

## Herbicides Tested

Herbicide IIa

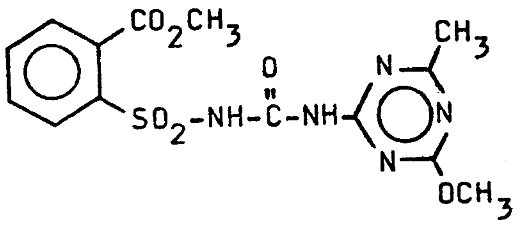

Herbicide IIb

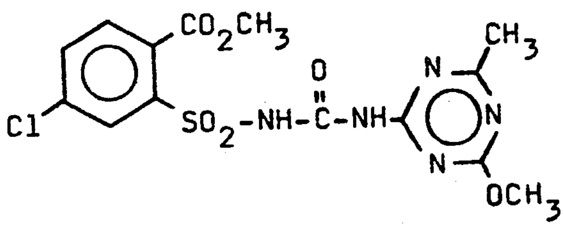

Herbicide IIc

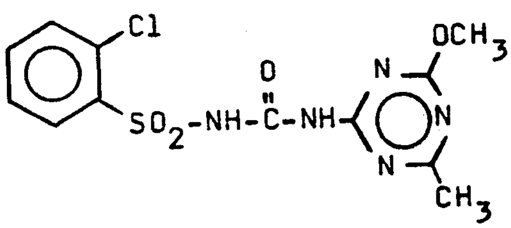

Herbicide IId

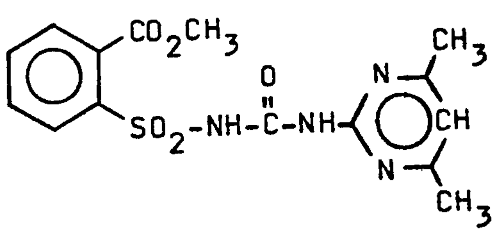

Herbicides Tested (continued)

Herbicide IIe

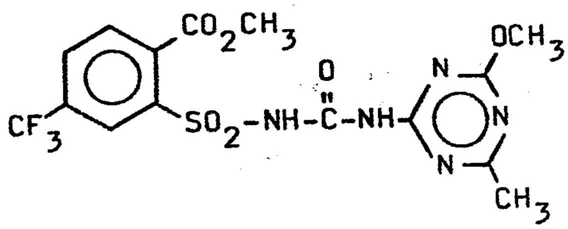

Herbicide IIf

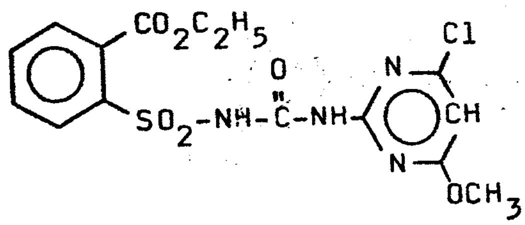

Postemergent Herbicide Antidote Tests

Crop plants, wheat, barley, corn, sorghum and soybeans used in the tests were grown in 5" or 6" plastic pots or in 10"x6"x3" Fiber Paks in steam sterilized soil: Terra-Lite Metro-Mix (350) Growing Medium (The Terra-Lite Metro-Mix Growing Medium, manufactured by Grace and Co., Cambridge, Mass.) in the greenhouse. The pots were watered with tap water and fertilized with Peters 20-20-20 general purpose fertilizer once a week. The herbicide-antidote treatments were made on the seedlings at the 1-3 leaf stages by a foliar spray. Application of the herbicide and antidote was made sequentially with single sprays of the herbicide or antidote formulation, the antidote formulation being sprayed first. Solutions of the herbicide and antidotes were made up separately in acetone-water-glycerine-surfactant (AGWT) at a volume rate of about 560 ℓ/ha.

Evaluation of the antidote action was made 2-3 weeks after the postemergent spray. The degree of injury caused by the herbicide/antidote applications and by the control applications (herbicide alone) was rated by visual evaluation and fresh weight measurement which was expressed as percent of untreated shoot fresh weight. Treatments with herbicide and solvent alone were also included in the tests. The results are given in Tables III to V. Many of the tests were repeated at different times of the year, accounting for the sometime divergent results. Although the growth stimulation (i.e., weight greater than 100% of check) observed in some of the tests may be due to the chemicals themselves, this effect may also be a result of lack of competition due to effective weed control.

## Table III

### Wheat Injury Rating
### Shoot Fresh Weight (% Ck)

| Antidote | Rate kg/ha | Herbicide IIa 0.125 kg/ha | | | | |
|---|---|---|---|---|---|---|
| None | - | 30.6 | 57.3 | 29.8 | 30.3 | 62.7 |
| 1 | 1.0 | 80.4 | | 94.8 | 84.0 | |
| | .250 | 85.4 | | 91.6 | 77.0 | 127.5 |
| | .063 | 61.2 | | 63.8 | 62.0 | |
| | .004 | | | 62.7 | | |

| Antidote | Rate kg/ha | Herbicide IIa 0.125 kg/ha | | | | |
|---|---|---|---|---|---|---|
| None | - | 73.5 | 60.7 | 57.5 | 136.6 | 41.6 |
| 1 | 1.0 | | | | | |
| | .250 | 186.3 | | 139.8 | 123.7 | |
| | .063 | | | | | |
| | .004 | | | | | |
| 2 | 1.0 | | 106.7 | | | 100.4 |
| | .250 | 236.3 | 80.8 | 157.7 | 278.9 | 90.1 |
| | .063 | | | | | 93.5 |
| 5 | .250 | | 71.5 | | | |
| 7 | 1.0 | | | | | 96.5 |
| | .250 | | 109.2 | | | 116.1 |
| | .063 | | | | | 106.3 |
| 8 | 1.0 | | | | | 86.2 |
| | .250 | | 63.5 | | | 103.1 |
| | .063 | | | | | 48.6 |
| 9 | .250 | | 96.4 | | | |
| 10 | 1.0 | | | | | 112.9 |
| | .250 | | | | | 95.2 |
| | .063 | | | | | 50.0 |
| 11 | .250 | | | 91.1 | | |
| 12 | 1.0 | | | 147.1 | | |
| | .250 | | | 96.4 | | |
| 13 | .500 | | | | 267.3 | |
| 14 | .500 | | | | 274.3 | |

Table III (continued)

Wheat Injury Rating
Shoot Fresh Weight (% Ck)

| Anti-dote | Rate kg/ha | Herbicide IIb 0.063 kg/ha | | | | |
|---|---|---|---|---|---|---|
| None | - | 17.8 | 34.3 | 17.0 | 32.4 | 59.2 |
| 1 | 1.0 | 76.7 | | | | |
| | .250 | 74.8 | 85.1 | 46.1 | 123.5 | 121.2 |
| | .063 | 36.4 | | | | |
| 2 | .250 | | | | | 158.6 |
| 3 | 1.0 | | | 49.1 | | |
| 5 | 1.0 | | | 36.7 | | |

| Anti-dote | Rate kg/ha | Herbicide IIb 0.063 kg/ha | | | |
|---|---|---|---|---|---|
| None | - | 12.1 | 18.2 | 11.7 | 11.1 |
| 1 | .250 | | 70.7 | 58.4 | |
| 2 | 1.0 | 96.1 | | | 55.9 |
| | .250 | 37.0 | 84.4 | 130.7 | 48.2 |
| | .063 | | | | 31.1 |
| 5 | .250 | 27.7 | | | |
| 7 | 1.0 | | | | 38.4 |
| | .250 | 72.5 | | | 57.1 |
| | .063 | | | | 30.1 |
| 9 | 1.0 | | | | 31.0 |
| | .250 | 17.5 | | | 25.4 |
| | .063 | | | | 20.9 |
| 10 | 1.0 | | | | 26.5 |
| | .250 | 53.9 | | | 37.1 |
| | .063 | | | | 22.2 |
| 12 | 1.0 | | 94.3 | | |
| | .250 | | 39.0 | | |
| 13 | .500 | | | 100.4 | |
| 14 | .500 | | | 88.7 | |

Table III (continued)

Wheat Injury Rating
Shoot Fresh Weight (% Ck)

| Anti-dote | Rate kg/ha | Herbicide IIc kg/ha 0.016 | 0.032 | 0.032 | 0.063 |
|---|---|---|---|---|---|
| None | - | 103.3 | 193.1 | 136.9 | 95.8 |
| 1 | .250 | 133.8 | 221.6 | 162.6 | |
| 2 | 1.0 | | | | 148.4 |
| | .250 | | | 150.8 | 128.8 |
| | .063 | | | | 111.1 |
| 7 | 1.0 | | | | 140.8 |
| | .250 | | | | 103.2 |
| | .063 | | | | 127.5 |
| 9 | 1.0 | | | | 143.0 |
| | .250 | | | | 138.4 |
| | .063 | | | | 131.0 |
| 10 | 1.0 | | | | 152.0 |
| | .250 | | | | 127.8 |
| 11 | .063 | | | | 122.0 |
| | .250 | | | 144.6 | |
| 12 | 1.0 | | | 164.7 | |
| | .250 | | | 174.2 | |

Table IV

Sorghum Injury Rating
Shoot Fresh Weight (% Ck)

| Anti-dote | Rate kg/ha | Herbicide IId kg/ha 0.016 | Herbicide IId 0.032 kg/ha | | | | |
|---|---|---|---|---|---|---|---|
| None | - | 61.5 | 25.7 | 33.7 | 39.2 | 24.0 | 110.4* |
| 1 | 2.0 | | 86.7 | | | | |
| | 1.0 | | 90.7 | | | | |
| | .500 | | 62.6 | | | | |
| | .250 | 84.8 | 52.3 | | 70.3 | 91.7 | |
| 2 | 1.0 | | | | | | 182.5 |
| | .250 | | | 79.5 | 71.4 | 92.7 | 187.1 |
| 7 | 1.0 | | | | | | 161.3 |
| | .250 | | | | | | 145.6 |
| 12 | 1.0 | | | | 113.2 | | |
| | .250 | | | | 58.2 | | |
| 13 | 1.0 | | | | | | 172.9 |
| | .500 | | | | | 103.2 | |
| | .250 | | | | | | 183.1 |
| 14 | 1.0 | | | | | | 204.3 |
| | .500 | | | | | 97.7 | |
| | .250 | | | | | | 151.2 |

* Plant treated with Herbicide IId alone had hormonal symptoms of injury.

Table IV (continued)

Sorghum Injury Rating
Shoot Fresh Weight (% Ck)

| Anti-dote | Rate kg/ha | Herbicide IIe kg/ha 0.016 | 0.016 | 0.004 | Herbicide IIf kg/ha 0.008 | 0.016 | 0.016 |
|---|---|---|---|---|---|---|---|
| None | - | 5.8 | 19.8 | 38.1 | 67.7 | 24.6 | 17.4 |
| 1 | .250 | 13.7 | | 67.9 | 85.5 | | |
| 2 | 1.0 | | 90.8 | | | 98.6 | 61.9 |
| | .250 | 7.9 | 43.7 | 85.2 | 88.4 | 62.2 | 57.3 |
| 5 | .250 | | | | | 42.8 | |
| 7 | 1.0 | | 92.0 | | | | 74.5 |
| | .250 | | 20.4 | | | 99.1 | 53.4 |
| 9 | .250 | | | | | 66.8 | |
| 10 | .250 | | | | | 57.5 | |
| 13 | 1.0 | | 70.5 | | | | 105.6 |
| | .250 | | 46.6 | | | | 51.3 |
| 14 | 1.0 | | 49.2 | | | | 79.8 |
| | .250 | | 29.4 | | | | 57.1 |

Table V

Soybean Injury Rating
Shoot Fresh Weight (% Ck)

| Anti-dote | Rate kg/ha | Herbicide IId kg/ha 0.001 | IIf kg/ha 0.008 | 0.016 |
|---|---|---|---|---|
| None | - | 24.1 | 113.7 | 116.4 |
| 1 | .250 | 42.6 | 131.5 | |
| 2 | 1.0 | | | 122.5 |
| | .250 | 28.3 | | 169.7 |
| 5 | .250 | | | 152.8 |
| 7 | .250 | | | 124.5 |
| 8 | .250 | | | 141.0 |
| 9 | .250 | | | 129.4 |
| 10 | .250 | | | 152.9 |
| 13 | .500 | 49.5 | | |
| 14 | .500 | 71.5 | | |

Antidote Protection by Seed Treatment

Wheat, sorghum or corn seeds were coated with 0.1 to 0.5% by seed weight with antidotes by placing a known weight of seed and antidote in a glass jar. A few drops of water were added and the jar was rotated until the seeds appeared evenly coated. After the seeds had been aerated thoroughly, they were planted in plastic pots or Fiber Paks in steam sterilized soil:Metro-Mix. Non-coated seeds were similarly planted. Sulfonylurea herbicide treatments were sprayed in AGWT at a volume rate of 560 ℓ/ha on the soil surface as a typical preemergence treatment (PRE). In the postemergence treatment (POST), the sulfonylurea herbicides were applied as foliar sprays to the plants 9 days after treating and planting seeds. Immediately after planting and treating in all but the postemergence treatments, the test containers were watered with a fine mist to activate the chemical treatments and initiate crop germination. The tests were then held in the greenhouse under standard greenhouse care until evaluation after 14-21 days. Shoot fresh weight is recorded and herbicide injury and/or antidoting is computed as percent of check plant shoot fresh weight. The results are recorded in Tables VI and VII.

Table VI

Wheat Injury Rating
Shoot Fresh Weight (% Ck)

| Herbicides | Rate kg/ha | Antidote 2: None | Antidote 2: Seed - 0.5% SW* |
|---|---|---|---|
| PRE | | | |
| IIa | 0.032 | 84.0 | 102.6 |
| IIb | 0.016 | 71.8 | 72.7 |
| | 0.004 | 84.5 | 115.0 |
| IIc | 0.063 | 82.7 | 107.9 |
| IId | 0.004 | 60.9 | 75.5 |
| POST | | | |
| IIa | 0.125 | 38.8 | 60.7 |
| | 0.125 | 52.4 | 74.8 |
| IIb | 0.063 | 5.7 | 24.1 |
| | 0.063 | 10.1 | 23.7 |
| | 0.016 | 35.5 | 61.3 |
| IIc | 0.063 | 80.0 | 100.9 |
| IId | 0.004 | 15.2 | 52.8 |
| | 0.004 | 12.3 | 35.5 |
| IIe | 0.016 | 13.5 | 41.0 |
| IIf | 0.063 | 29.0 | 39.8 |

* %SW = percent seed weight

Table VII

Sorghum Injury Rating
Shoot Fresh Weight (% Ck)

| Herbicides | Rate kg/ha | Antidote 2: None | Antidote 2: Seed - 0.5% SW* |
|---|---|---|---|
| PRE | | | |
| IIa | 0.008 | 52 | 94 |
| IIb | 0.032 | 39 | 85 |
| IIc | 0.032 | 10 | 96 |
| | 0.008 | 63 | 91 |
| IIe | 0.008 | 0 | 79 |
| IIf | 0.008 | 76 | 95 |
| POST | | | |
| IIa | 0.032 | 7 | 14 |
| | 0.008 | 11 | 39 |
| IIb | 0.032 | 5 | 17 |
| | 0.008 | 6 | 18 |
| IIc | 0.032 | 13 | 39 |
| IIe | 0.032 | 7 | 9 |
| | 0.008 | 4 | 11 |
| IIf | 0.032 | 9 | 29 |
| | 0.008 | 19 | 36 |

* %SW = percent seed weight

Postemergence Field Test

Wheat, Arthur 71 was planted in the field with rows of weeds also planted. Antidote 1 and herbicides were applied postemergence when the wheat was fully tillered and about 4-6 inches tall. The treatment components were dissolved in water which had 0.1% Citowett added. The water-Citowett solvent was sprayed on similar plots for comparison.

The response of wheat and weeds to the treatment was evaluated 20 days after spraying by visual observations as recorded in Table VIII. The yield of wheat seeds at maturity was also recorded. Herbicide injury was rated on a scale of 0 to 100 in which 0 indicated no injury and 100 indicated that the plants were dead.

Table VIII

Evaluation of herbicidal candidates and combinations for post-emergence weed control in winter wheat in the field.

Injury Rating[1]
(20 days after treatment)

| Herbicide | Rate kg/ha | Antidote 1 Post kg/ha | Wheat | Yellow Rocket | Mouse ear Chickweed | Wild Garlic |
|---|---|---|---|---|---|---|
| None | - | - | 0 | 0 | 0 | 0 |
| IIa | .004 | 0 | 0 | 70 | 50 | 7 |
| | .008 | 0 | 0 | 72 | 55 | 5 |
| | .016 | 0 | 0 | 77 | 57 | 20 |
| | .031 | 0 | 10 | 80 | 75 | 40 |
| | .062 | 0 | 12.5 | 80 | 65 | 35 |
| | .031 | .031 | 0 | 85 | 65 | 25 |
| | .062 | .031 | 7.5 | 80 | 65 | 35 |
| IIb | .002 | 0 | 0 | 55 | 30 | 10 |
| | .004 | 0 | 15 | 60 | 65 | 15 |
| | .008 | 0 | 25 | 70 | 60 | 5 |
| | .016 | 0 | 25 | 80 | 60 | 20 |
| | .002 | .031 | 0 | 60 | 50 | 0 |
| | .004 | .031 | 5 | 70 | 60 | 15 |
| | .008 | .031 | 0 | 70 | 60 | 5 |
| | .016 | .031 | 5 | 80 | 65 | 0 |
| IIa+IIb | .008+.002 | 0 | 5 | 75 | 60 | 10 |
| | .008+.004 | 0 | 5 | 70 | 65 | 5 |
| | .008+.008 | 0 | 17 | 75 | 65 | 15 |
| | .008+.002 | .031 | 0 | 80 | 65 | 20 |
| | .008+.004 | .031 | 0 | 80 | 60 | 5 |
| | .008+.008 | .031 | 0 | 80 | 60 | 20 |
| IIb+IIc | .002+.008 | 0 | 0 | 75 | 50 | 20 |
| | .004+.008 | 0 | 5 | 80 | 50 | 20 |
| | .008+.008 | 0 | 25 | 80 | 60 | 15 |
| | .002+.008 | .031 | 2.5 | 80 | 45 | 15 |
| | .004+.008 | .031 | 0 | 80 | 55 | 15 |
| | .008+.008 | .031 | 5 | 85 | 65 | 5 |

[1] Injury rating 0=no effect 100=complete kill

Table VIII (continued)

| Herbicide | Rate kg/ha | Anti-dote 1 Post kg/ha | Injury Rating[1] (20 days after treatment) Red Sorrell | Blue-Grass | Thistle | Yield (g/plot) |
|---|---|---|---|---|---|---|
| None | - | - | 0 | 0 | - | 429 |
| IIb | .002 | 0 | 60 | 0 | 0 | 408 |
| | .004 | 0 | 75 | 0 | 0 | 438 |
| | .008 | 0 | 75 | - | 0 | 470 |
| | .016 | 0 | 80 | 0 | - | 482 |
| | .002 | .031 | 70 | 0 | 5 | 526 |
| | .004 | .031 | 75 | 0 | - | 466 |
| | .008 | .031 | 85 | 0 | - | 569 |
| | .016 | .031 | 80 | 0 | - | 528 |
| IIa+IIb | .008+.002 | 0 | 75 | - | - | 425 |
| | .008+.004 | 0 | 80 | - | - | 476 |
| | .008+.008 | 0 | 80 | - | 30 | 472 |
| | .008+.002 | .031 | 75 | 0 | 10 | 483 |
| | .008+.004 | .031 | 80 | 0 | - | 516 |
| | .008+.008 | .031 | 80 | - | - | 486 |
| IIb+IIc | .002+.008 | 0 | 70 | - | 15 | 410 |
| | .004+.008 | 0 | 85 | - | - | 531 |
| | .008+.008 | 0 | 80 | - | 40 | 506 |
| | .002+.008 | .031 | 70 | - | - | 526 |
| | .004+.008 | .031 | 85 | - | - | 556 |
| | .008+.008 | .031 | 85 | - | - | 520 |

[1] Injury rating 0=no effect 100=complete kill

Claims:

1. A compound of the formula:

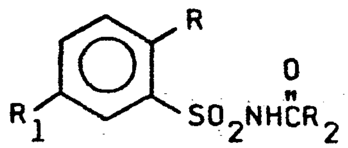 or 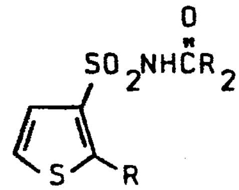

I II

where

R is $CO_2CH_3$ or $CO_2C_2H_5$;

$R_1$ is H, Cl or $CF_3$;

$R_2$ is $C_1$-$C_3$ alkoxy or $NR_3R_4$;

$R_3$ is H, $OCH_3$, $C_1$-$C_4$ alkyl, cyclohexyl, $C_1$-$C_4$ alkyl substituted with 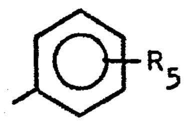, $C_1$-$C_4$ alkyl substituted with $C_1$-$C_2$ alkoxy or $C_1$-$C_4$ alkyl substituted with ethoxyethoxy;

$R_4$ is H or $C_1$-$C_2$ alkyl;

$R_5$ is H, Cl or $OCH_3$;

provided that, in a compound of Formula I, when R is $CO_2CH_3$ and $R_2$ is $C_1$-$C_3$ alkoxy, then $R_1$ is other than H; and agriculturally suitable salts.

2. A compound of Claim 1 where $R_1$ is H.

3. A compound of Claim 1 where $R_2$ is $NR_3R_4$.

4. A compound of Claim 3 where $R_1$ is H.

5. A compound of Claim 4 where $R_3$ is H or $C_1$-$C_4$ alkyl optionally substituted with unsubstituted phenyl or $C_1$-$C_2$ alkoxy.

6. A compound of Claim 4 where $R_4$ is H.

7. A compound of Claim 3 where $R_3$ is other than $C_4$-alkyl.

8. The compound of Claim 1 which is 2-[(aminocarbonyl)aminosulfonyl]benzoic acid, methyl ester and agriculturally suitable salts thereof.

9. The compound of Claim 1 which is 3-[(aminocarbonyl)aminosulfonyl]-2-thiophenecarboxylic acid, methyl ester.

10. An agricultural composition comprising a non-phytotoxic antidotally effective amount of an antidotal compound of any of the preceding claims and at least one of (a) a surfactant and (b) a solid or liquid diluent.

11. Seed having a coating comprising a non-phytotoxic antidotally effective amount of a compound of any of claims 1 to 9.

12. The seed of claim 11 comprising from 0.1 to 0.5% of said compound, based on the seed weight.

13. A method of protecting crop plants from injury due to an herbicial sulfonylurea compound of the formula

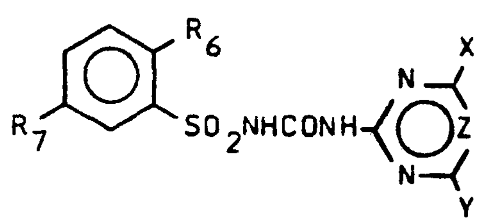

where

$R_6$ is $CO_2R_8$ or Cl;

$R_7$ is H, Cl or $CF_3$;

$R_8$ is $C_1$-$C_3$ alkyl;

X is $CH_3$ or $OCH_3$;

Y is $CH_3$, $OCH_3$ or Cl; and

Z is CH or N;

provided that

(a) when Y is Cl, then Z is CH; and

(b) when $R_6$ is Cl, then $R_7$ is H, X is $CH_3$, Y is $OCH_3$ and Z is N;

and their agriculturally suitable salts thereof, comprising combining application of an herbicidally effective amount of said herbicidal compound to the

locus of said crop plants with application of a non-phytotoxic antidotally effective amount of an antidotal compound of the formulas

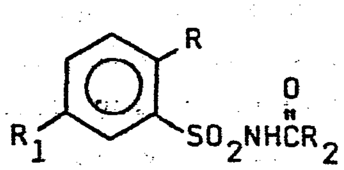

or

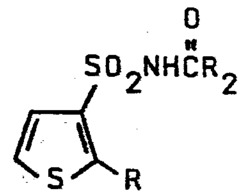

I

II

where

R is $CO_2CH_3$ or $CO_2C_2H_5$;

$R_1$ is H, Cl on $CF_3$;

$R_2$ is $C_1$-$C_3$ alkoxy or $NR_3R_4$;

$R_3$ is H, $OCH_3$, $C_1$-$C_4$ alkyl, cyclohexyl, $C_1$-$C_4$ alkyl substituted with 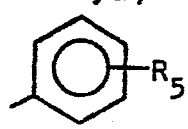, $C_1$-$C_4$ alkyl substituted with $C_1$-$C_2$ alkoxy or $C_1$-$C_4$ alkyl substituted with ethoxyethoxy;

$R_4$ is H or $C_1$-$C_2$ alkyl;

$R_5$ is H, Cl or $OCH_3$;

and their agriculturally suitable salts, to the crop seed, to the crop plant or to the locus of the crop plant.

14. A method of claim 13 wherein said antidotal compound is as defined in any of claims 2 to 9, except that the proviso in claim 1 does not apply.

15. A method of Claim 13 or 14 where the crop is selected from wheat, barley, corn, sorghum and soybean.

16. A method of Claim 13 or 14 where the crop is wheat.

17. A herbicidal composition comprising an herbicidally effective amount of an herbicidal sulfonylurea compound as defined in claim 13

and a non-phytotoxic antidotally effective amount of an antidotal compound of the formulas (I) or (II) as defined in claim 13.

18. The composition of claim 15 wherein said antidotal compound is as defined in any of claims 2 to 9, except that the proviso in claim 1 does not apply.

19. A method for the preparation of a compound of formula (I) or (II) as defined in claim 1 which comprises

(a) reacting an isocyanate of formula

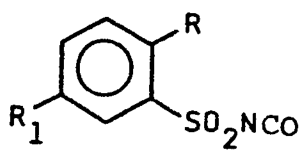

or

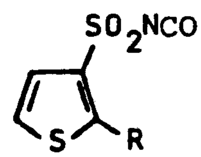

(IV)

(V)

wherein R and $R_1$ are as defined in claim 1, with an amine or alcohol of formula

$$HR_2$$

wherein $R_2$ is as defined in claim 1; or

(b) reacting a sulfonamide of formula

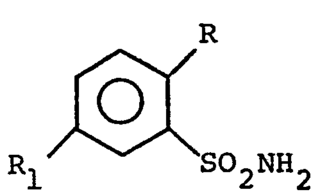

or

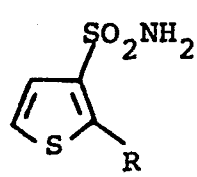

wherein R and $R_1$ are as defined in claim 1, with a $C_1$-$C_3$ alkyl chloroformate, to prepare a compound of claim 1 wherein $R_2$ is $C_1$-$C_3$ alkoxy; or

(c) reacting a sulfonamide as defined in (b) above with

$$OCNR_4$$

wherein $R_4$ is $C_1$-$C_2$ alkyl.

Claims:

1. A method of protecting crop plants from injury due to an herbicial sulfonylurea compound of the formula

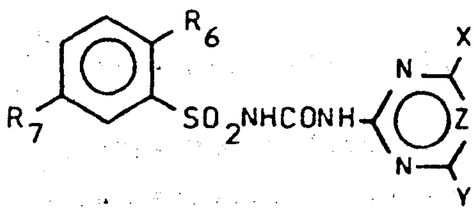

where

$R_6$ is $CO_2R_8$ or Cl;
$R_7$ is H, Cl or $CF_3$;
$R_8$ is $C_1$-$C_3$ alkyl;
X is $CH_3$ or $OCH_3$;
Y is $CH_3$, $OCH_3$ or Cl; and
Z is CH or N;

provided that

(a) when Y is Cl, then Z is CH; and
(b) when $R_6$ is Cl, then $R_7$ is H, X is $CH_3$, Y is $OCH_3$ and Z is N;

and their agriculturally suitable salts thereof, comprising combining application of an herbicidally effective amount of said herbicidal compound to the locus of said crop plants with application of a non-phytotoxic antidotally effective amount of an antidotal compound of the formulas

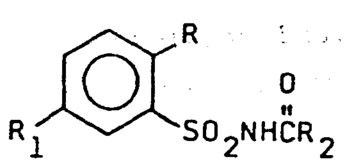

or

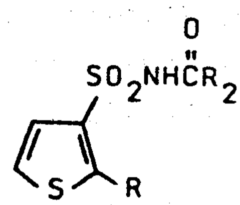

I

II

where

R is $CO_2CH_3$ or $CO_2C_2H_5$;

$R_1$ is H, Cl on $CF_3$;

$R_2$ is $C_1$-$C_3$ alkoxy or $NR_3R_4$;

$R_3$ is H, $OCH_3$, $C_1$-$C_4$ alkyl, cyclohexyl,

$C_1$-$C_4$ alkyl substituted with 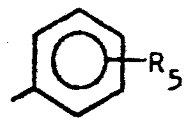,

$C_1$-$C_4$ alkyl substituted with $C_1$-$C_2$ alkoxy or $C_1$-$C_4$ alkyl substituted with ethoxyethoxy;

$R_4$ is H or $C_1$-$C_2$ alkyl;

$R_5$ is H, Cl or $OCH_3$;

and their agriculturally suitable salts, to the crop seed, to the crop plant or to the locus of the crop plant.

2. A method of Claim 1 where $R_1$ is H.

3. A method of Claim 1 where $R_2$ is $NR_3R_4$.

4. A method of Claim 3 where $R_1$ is H.

5. A method of Claim 4 where $R_3$ is H or $C_1$-$C_4$ alkyl optionally substituted with unsubstituted phenyl or $C_1$-$C_2$ alkoxy.

6. A method of Claim 4 where $R_4$ is H.

7. A method of Claim 3 where $R_3$ is other than $C_4$-alkyl.

8. The method of Claim 1 wherein said antidotal compound is selected from 2-[(aminocarbonyl)aminosulfonyl]benzoic acid, methyl ester and agriculturally suitable salts thereof; and 3-[(aminocarbonyl)-aminosulfonyl]-2-thiophenecarboxylic acid, methyl ester.

9. A method of any of claims 1 to 8 where the crop is selected from wheat, barley, corn, sorghum and soybean.

10. Seed having a coating comprising a non-phytotoxic antidotally effective amount of a compound of formula (I) or (II) as defined in any of claims 1 to 8.

11. A herbicidal composition comprising an herbicidally effective amount of an herbicidal sulfonylurea compound as defined in claim 1 and a non-phytotoxic antidotally effective amount of an antidotal compound of the formulas (I) or (II) as defined in any of claims 1 to 8.

12. A method for the preparation of a compound of formula (I) or (II) as defined in claim 1, provided that, in a compound of Formula I, when R is $CO_2CH_3$ and $R_2$ is $C_1$-$C_3$ alkoxy, then $R_1$ is other than H, which comprises

(a) reacting an isocyanate of formula

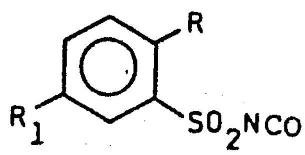

or

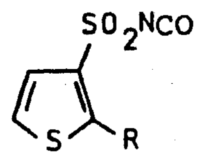

(IV) (V)

wherein R and $R_1$ are as defined in claim 1, with an amine or alcohol of formula

$$HR_2$$

wherein $R_2$ is as defined in claim 1; or

(b) reacting a sulfonamide of formula

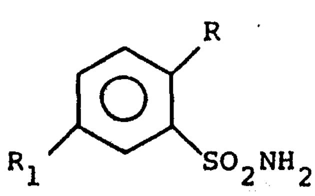

or

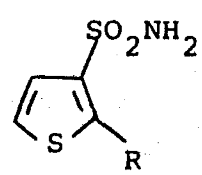

wherein R and $R_1$ are as defined in claim 1, with a $C_1$-$C_3$ alkyl chloroformate, to prepare a compound of claim 1 wherein $R_2$ is $C_1$-$C_3$ alkoxy; or

(c) reacting a sulfonamide as defined in (b) above with

$$OCNR_4$$

wherein $R_4$ is $C_1$-$C_2$ alkyl.